# EUROPEAN PATENT SPECIFICATION

(11) **EP 0 200 341 B1**
(45) Date of publication and mention of the grant of the patent: **05.01.1994**
(21) Application number: 86302112.7
(22) Date of filing: 21.03.1986
(51) Int. Cl.: C12N 15/18, C12N 5/00, C07K 13/00, C12P 21/02

(54) **Nucleic acid encoding TGF-beta and its uses**
TGF-beta codierende Nucleinsäure und deren Verwendungen
Acide nucléique codant de TGF-bêta et ses utilisations

(30) Priority: 22.03.1985 US 715142
(43) Date of publication of application: 10.12.1986
(73) Proprietor: GENENTECH, INC., South San Francisco California 94080 (US)
(72) Inventor: Derynck, Rik Michel Andre, Burlingame California 94010 (US); Goeddel, David Vannorman, Hillsborough California 94010 (US)
(74) Representative: Armitage, Ian Michael

(56) References cited:
- EP-A- 0 154 434
- Cell vol. 38, no. 1, August 1984 (Cambridge, Mass) R Derynck et al.: "Human Transforming Growth Factor-alpha: Precursor Structure and Expression in E. coli". pages 287-297
- Proceedings of the National Academy of Sciences of the USA, vol. 80, no. 12, June 1983 (Baltimore, USA) C A Frolik et al.: "Purification and initial characterization of a type Beta transforming growth factor from human placenta". pages 3676-3680
- Virology, vol. 108, no.2, January 30, 1981 (New York) P L Kaplan et al.: "Simian Virus 40 Inducos the Production of a Polypeptide Transforming Factor(s)" pages 484-490
- PNAS 80 (1983) 7461-5
- J Biol Chem 262 (1987) 12127-31
- "From Genes to Clones" by E L Winnacker, Preface pp. v -vii, VCH 1987
- Mol. Cell. Biol. 7 (1987) 3418-27

## Description

Peptides which can induce the reversible phenotypic transformation of mammalian cells in culture have been given the name transforming growth factor (TGF)^{1,2}. Type α TGF competes with epidermal growth factor (EGF) for binding to the same cell surface receptor³. A 50 amino acid TGF-α species has been purified and shown to share sequence homology with EGF⁴. TGF-α is synthesized by various transformed cell lines, but its synthesis has not yet been demonstrated in normal tissue of nonembryonic origin^{3,5,6}. The 50 amino acid TGF-α is initially synthesized as part of a 160 amino acid precursor molecule which undergoes N- and C-terminal proteolytic processing to yield the mature peptide^{7,8}. The detection of TGF-α species with apparently higher molecular weights^{1,2,9} might be due to variable processing of the 160 amino acid precursor.

Type β TGF activity has been isolated from tumor cells as well as many normal tissues^{10,11}, including kidney¹², placenta¹³ and blood platelets^{14,15}. TGF-β is present in platelets, which also contain platelet-derived growth factor (PDGF) and an EGF-like peptide¹⁶. Bovine TGF-β has been demonstrated to accelerate wound healing in rats¹⁷. Treatment of NRK fibroblasts with TGF-β does, however, result in an increase in the number of membrane receptors for EGF²⁰. This observation is in agreement with the known ability of TGF-β to greatly potentiate the activity of EGF and TGF-α on these cells^{10,11}. Moreover, TGF-β alone can induce AKR-2B fibroblasts to form colonies in soft agar²¹. The elevated levels of TGF-β secreted by some transformed cells²² suggest that this growth regulator could play a role in malignant transformation.

In addition to its ability to stimulate cell proliferation, TGF-β has recently been demonstrated to inhibit the anchorage-dependent growth of a variety of human cancer cell lines¹³. It is now thought that TGF-β may be identical or very similar to a growth inhibitor isolated from African green monkey (BSC-1) cells²⁴. Whether TGF-α acts to stimulate or inhibit the growth of a particular cell type line appears to depend on many variables, including the physiological condition of the cell and the presence of additional growth factors.

Bovine TGF-β has been purified to sequenceable grade. The first 15 amino-terminal residues of the mature protein were found to be Ala-Leu-Asp-Thr-Asn-Tyr-CMC-Phe-Ser-Ser-Thr-Gly-Lys-Asn-CMC-, wherein CMC is S-carboxymethyl cysteine representing cysteine or half-cystine residues.

Human TGF-β from human placenta and platelets has been purified to the same degree. Placenta TGF-β was reported to have the following amino terminal sequence: 
Ala-Leu-Asp-Thr-Asn-Tyr-CMC-Phe-(Ser-Ser)-Thr-Glu-Lys-Asn-CMC-Val-X-
Gln-Leu-Tyr-Ile-Asp-Phe-X-(Lys)-Asp-Leu-Gly-,
wherein X was undetermined and CMC is as defined above. Platelet TGF-β was reported as the amino terminal sequence Ala-Leu-Asp-Thr-Asn-Tyr-X-Phe-Ser, wherein CMC and X are as defined above.

Human TGF-β was reported to be composed of two polypeptide chains of very similar molecular weight (Mᵣ=12,500) which are maintained in covalent association by disulfide bonds. The disulfide bonds were considered likely to play an important role in conferring structure on the TGF-β molecule.

A method for making TGF-β is required that dispenses with the need for biological materials from humans as a starting material. This will contribute significantly to assurances that no infectious contaminants, e.g. HTLV-III or hepatitis viruses are able to enter the product preparation stream. Recombinant synthesis of TGF-β would accomplish this objective. However, in order to do so, nucleic acid encoding TGF-β is needed for the preparation of vectors to be used in such recombinant synthesis of TGF-β. Such nucleic acid also is needed for the diagnosis of TGF-β messenger and genomic DNA in tissue samples.

### SUMMARY

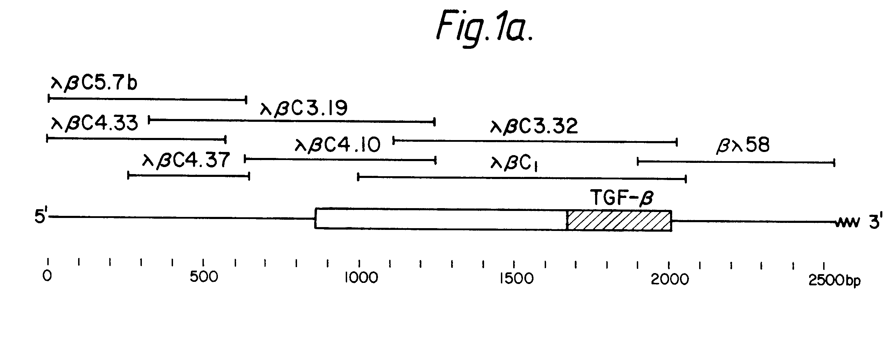

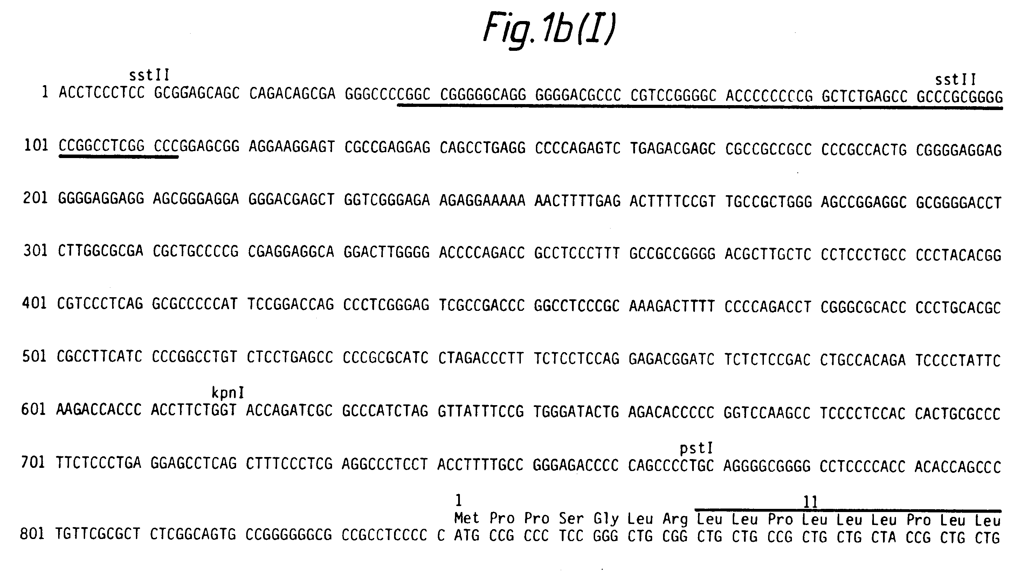

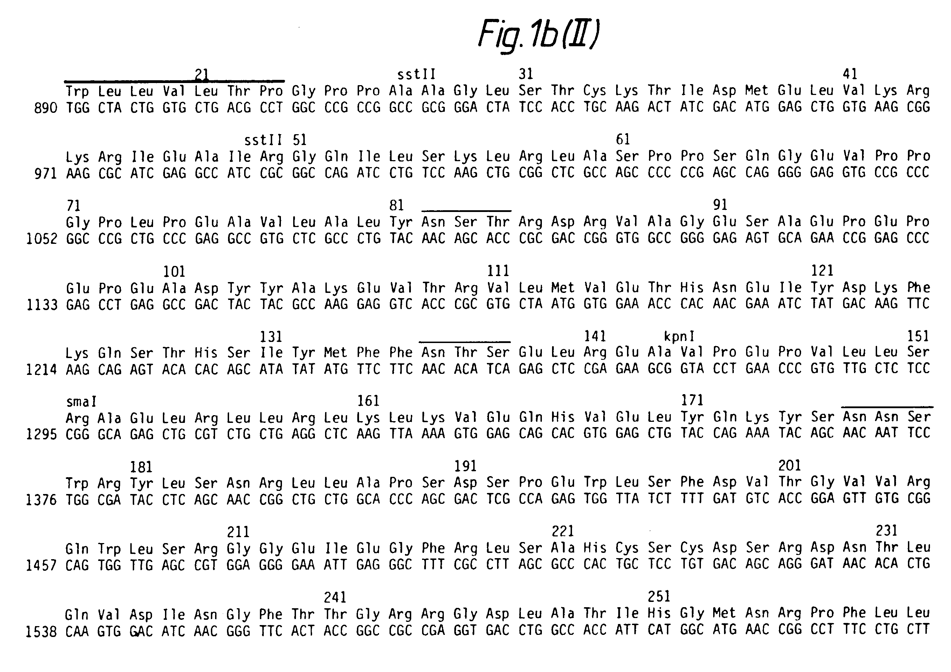

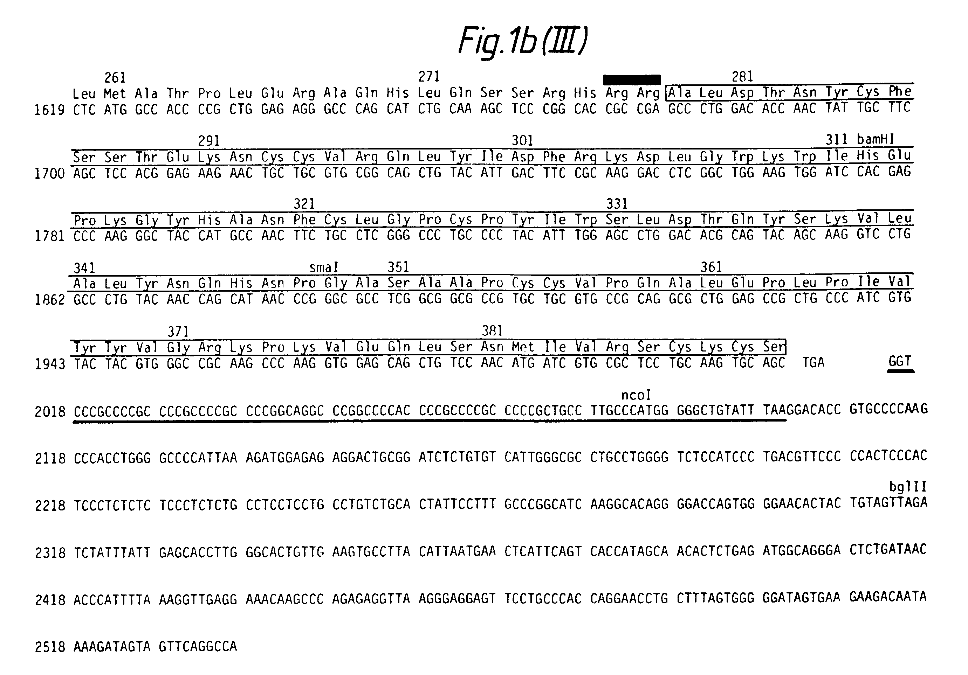

In accordance with this invention, the foregoing objects are achieved by a method comprising (a) constructing a vector which includes nucleic acid encoding pre TGF-β having the amino acid sequence shown in Figure 1b, or a mutant sequence thereof (b) transforming a host vertebrate cell with the vector, (c) culturing the transformed cell, and (d) recovering mature TGF-β or mutant thereof from the culture medium, said TGF-β or mutant thereof having anchorage independent growth-promoting biological activity and cross-reacting with antisera raised against native TGF-β.

Nucleic acid that encodes TGF-β having the amino acid sequence shown in Figure 1b is provided herein. It is useful in constructing the vectors.

This nucleic acid or a nucleic acid capable of hybridizing therewith can be labelled and used in diagnostic assays for DNA or mRNA encoding TGF-β or related proteins.

The preparation of TGF-β derivatives by recombinant methods is made possible by knowledge of the TGF-β coding sequence disclosed herein. These derivatives include silent and expressed mutants in the nucleic acid encoding TGF-β.

Silent mutants involve the substitution of one degenerate codon for another where both codons code for the same amino acid, but which substitution could exert a salutory effect on TGF-β yield in recombinant culture, e.g. by modifying in the secondary structure of TGF-β mRNA, and which salutory substitution is identified by screening TGF-β yields from transformants.

Expressed TGF-β mutants fall into one or more of three classes: Deletions, substitutions or insertions. Deletions are characterized by the elimination of amino acid residues without the insertion of a replacement residue. Deletion-mutated TGF-β DNA is useful in making TGF-β fragments, for example where it is desired to delete an immune epitope.

Substitution mutations are those in which one amino acid residue has been replaced by another. Such mutations are extremely difficult to make by methods other than recombinant synthesis, especially substitutions targeted for the interior of the primary amino acid sequence. They are useful in modifying the biological activity of TGF-β.

Insertional mutants are those in which one or more residues are placed into or at either end of the TGF-β nucleic acid. Fusions typically are a species of insertional mutant in which the insertion occurs at the carboxyl or amino terminal residues of TGF-β. TGF-β fusions with bacterial or other immunogenic proteins are useful for raising antibodies against TGF-β or its predetermined fragments.

### BRIEF DESCRIPTION OF THE DRAWINGS

Fig. 1a is a schematic diagram of the TGF-β mRNA showing the boxed coding sequence. The 112 amino acid TGF-β (dashed) is encoded by the 3' end of the coding sequence. The sequenced cDNA inserts of λβC1, 3.19, 3.32, 4.10, 4.33, 4.37 and 5.7b (described infra) and the genomic DNA sequence for the 3' untranslated region are aligned above the diagram.

Fig. 1b depicts the sequence and deduced amino acid sequence of the preTGF-β cDNA, determined from the several overlapping cDNAs and the genomic 3' region. The 5' terminal region which could be folded into stable hairpin loops is underlined. The 5' proximal ATG which does not function as initiator codon and the stop codon 19 triplets downstream are boxed. The preTGF-β cDNA encodes a 354 amino acid protein, of which the C-terminal 112 amino acids (boxed) encode mature TGF-β. An underlined Arg-Arg dipeptide precedes the proteolytic cleavage site for release of TGF-β. Three potential N-glycosylation sites in preTGF-β are overlined. The stop codon is followed by the underlined G-C rich sequence and a downstream TATA-like sequence. The AATAAA polyadenylation signal and TTCAGGCC sequence preceding the putative polyadenylation site are also underlined.

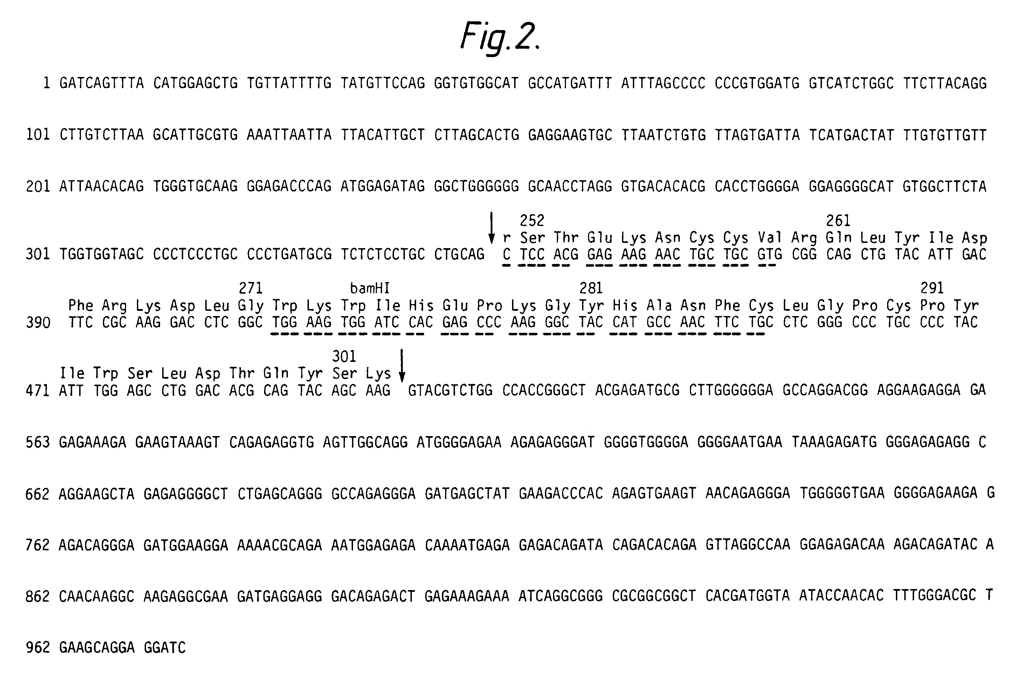

Fig. 2 depicts a genomic fragment encoding a TGF-β exon and its deduced amino acid sequence. Arrows show the mRNA processing sites (intron-exon junctions). The residue numbers correspond to Fig. 1b.

### DETAILED DESCRIPTION

TGF-β has proven to be extremely difficult to synthesize in recombinant cell culture while retaining its growth-promoting activity. As can be seen from Fig. 1 the mature TGF-β amino acid sequence contains a large number of cysteine residues (9), at least some of which apparently are involved in interchain crosslinking in forming the homodimeric TGF-β which is recovered from natural sources. Furthermore, TGF-β is expressed as a precursor molecule having a large amino terminal region not containing the recognizable NH₂-terminal signal peptide sequence typical of most secreted proteins, even though TGF-β normally appears to some degree in the extracellular medium. Without limiting the disclosure herein to any particular theory, this amino-terminal region may contain a plurality of transmembrane regions. However, we have been able to transform eukaryotic cells to express heterologous TGF-β, notwithstanding the anticipated difficulty in properly processing the primary translation product in recombinant culture.

This invention is directed to recombinant synthesis of TGF-β, which is defined as inclusive of biologically active preTGF-β having the Fig. 1b sequence, mature TGF-β, polypeptide fragments thereof and insertion, substitution and/or deletion mutants of such preTGF-β, mature TGF-β or polypeptide fragments.

Biologically active TGF-β is defined as being capable of inducing EGF-potentiated anchorage independent growth of target cell lines⁸¹ and/or growth inhibition of neoplastic cell lines²³. Anchorage independent growth refers to the ability of TGF-β and EGF treated non-neoplastic target cells to form colonies in soft agar, a characteristic ascribed to transformation of the cells (hence the name transforming growth factor). This is not to say that TGF-β will "cause" cancer because it is now known that many normal cells express TGF-β. Paradoxically, TGF-β also is known to inhibit the growth of certain neoplastic cells such as A549.

Biological activity for the purposes herein also generally includes the ability to cross-react with antisera raised against native TGF-β. Native TGF-β is that which is obtained from platelets or other natural sources. Immunological cross-reactivity is a measure of a single active epitope and not necessarily of active TGF-β domains involved in inducing anchorage-independent growth or target cells. However, immunologically cross-reactive proteins per se are not biologically active as defined herein unless they also exert growth-affecting activity. Of course, TGF-β which is capable of inducing anchorage independent growth frequently will exhibit immunological cross-reactivity with antisera raised against the native molecule as a corollary to maintenance of proper conformation.

The Fig. 1 nucleotide sequence was obtained by an analysis of several overlapping cDNAs and gene fragments, leading to the determination of a continuous sequence of 2439 base pairs corresponding to the TGF-β precursor mRNA. An initiator ATG is located 952 nucleotides from the 5' end and establishes a coding sequence of 1,062 nucleotides, thus coding for a 354 amino acid long polypeptide. Several areas within the cDNA sequence have an exceptionally high G-C content. The initiator ATG is flanked by two G-C rich areas of approximately 200 bp each. In addition, several regions of the cDNA, particularly the 5'-terminus, have regions with greater than 80 percent G-C content. The location of these G-C rich regions coincides with the areas in which the many cDNA cloning artifacts occurred and where partial length cDNAs were obtained.

Several structural features favor the assignment of the ATG at position 953 as the start codon for preTGF-β. First, the sequence context at this position is in reasonably agreement with the proposed initiation codon consensus sequence G/A CCATGG³⁵. Second, even though this ATG is preceded by an open reading frame for about 850 bp, no other ATG can be found in the same reading frame and an in-phase stop codon is present at position 85. Thirdly, computation of the most likely used reading frame, based upon the purine-pyrimidine distribution as described by Shepherd³⁶, shows that the coding sequence starting from the initiator ATG is indeed the most probable reading frame, while preceding this ATG a different reading frame is selected as the most likely one. Fourth, this ATG is localized within a 40 nucleotide region of relatively low G-C content (∼50 percent), which might favor accessibility to the ribosome, especially since it is localized within a larger G-C rich region. There is only a single other potential initiation codon in the 5' untranslated sequence (position 842). However, this ATG falls within a different reading frame, is followed 19 triplets later by a stop codon, and is located in a very G-C rich region.

The 5' untranslated region of the TGF-β mRNA is at least 952 nucleotides long and contains a 61 nucleotide long sequence (192 to 252) consisting almost exclusively of purines. The biological relevance of this exceptionally long 5' untranslated region of high G-C content is unknown, but it is similar to the structural organization of c-myc mRNA. However, there is no striking sequence homology between these two sequences. The long 5' untranslated region of c-myc has been hypothesized to have a functional significance³⁷. The G-C rich 5'-proximal part of the 5' untranslated sequence of human c-myc mRNA has several regions which could form stable hairpin loops. Likewise, the first 120 bp of the untranslated preTGF-β cDNA can theoretically be folded into hairpin loop structures with a calculated stability of -91 kcal. The long 5' untranslated sequence and the potentially stable hairpin loop structures could play a role in mRNA stability or in the regulation of transcription. Accordingly, this region can be deleted and substituted for by other 5' untranslated sequences, e.g. from viral proteins, in order to identify structures that may improve TGF-β yields from recombinant cell culture.

The stop codon at residue 2015 is immediately followed by a remarkable, G-C rich sequence of 75 nucleotides (Fig. 1b). This sequence consists of multiple repeats of CCGCC and ends with GGGGGC. The peculiar nature of this sequence is probably responsible for the fact that the 3' untranslated end of the mRNA could not be cloned as a cDNA sequence, perhaps due to the inability of the E. coli DNA polymerase I to use this sequence as a template for the second strand cDNA synthesis. Repeat sequences of a similar nature have been found in the promoter regions of the genes for human dihydrofolate reductase, human transferrin receptor, human adenosine deaminase, and Herpesvirus thymidine kinase⁴⁰. In the latter case, McKnight et al.⁴⁰ have shown that these structural elements are of major importance for the transcription efficiency. In addition, it has been shown that the promoter specific transcriptional factor Spl binds to such sequences in the SV40 early promoter region and in a related monkey promoter^{41,42}. In all of these cases the G-C rich repeats are followed closely by the Goldberg-Hogness TATA sequence. In the case of preTGF-β, however, these sequences are located in the 3' untranslated region of the gene, but are interestingly also followed by a TATA-like sequence. No evidence that this region could function as a promoter is available. The preTGF-β gene sequence has the hexanucleotide AATAAA about 500 nucleotides downstream from the stop codon. This sequence, which usually precedes the site of polyadenylation by 11 to 30 bases³², probably functions as the preTGF-β mRNA polyadenylation signal, since this would be in agreement with the size of preTGF-β mRNA estimated from Northern hybridizations, and since 3' untranslated regions rarely contain intervening sequences. Benoist et al.⁴³ have proposed a consensus sequence TTCACTGC which follows the AATAAA closely and immediately precedes the polyA-tail. A similar sequence, TTCAGGCC, follows the AATAAA sequence in the 3' untranslated region of the preTGF-β mRNA, providing further support for the assignment of the polyadenylation site at position 2530 (Fig. 1b).

PreTGF-β is a polypeptide of 354 amino acids (Fig. 1b). Comparison of this sequence with the previously determined NH₂-terminus of mature TGF-β shows that TGF-β constitutes the C-terminal 112 amino acids of preTGF-β. The mature TGF-β monomer is cleaved from the precursor at the Arg-Arg dipeptide immediately preceding the mature TGF-β NH₂-terminus. Similar proteolytic cleavage sites have been found in several other polypeptide precursor sequences, including preproenkephalin^{44,45}, the calcitonin precursor⁴⁶, and corticotropin-β-lipotropin precursor⁴⁷. Determination of the hydrophobicity profile by the method of Kyte and Doolittle⁴⁸ predicts that this Arg-Arg sequence is located within a hydrophilic region which would make it accessible to a trypsin-like peptidase. Post-translational cleavage of the 354 amino acid precursor gives rise to the mature TGF-β monomer. The disposition of the presequence is not known but may give rise to other biologically active peptides. The TGF-β precursor contains several pairs of basic residues (Fig. 1b) which could also undergo post-translation cleavage and give rise to separate polypeptide entities. However, mature TGF-β contains two Arg-Lys dipeptides which apparently are not cleaved. As shown in Fig. 1b, preTGF-β precursor contains three potential N-glycosylation sites, Asn-X-Ser or Thr (49, Fig. 1b). None of these are localized within mature TGF-β. Accordingly, a method is provided whereby mature TGF-β is purified free of glycoproteins by adsorbing the glycoproteins on immobilized lectins and eluting TGF-β with the unadsorbed fraction.

The sequence for human TGF-β was determined by direct amino acid sequence analysis and by deduction from the TGF-β cDNA. The sequence of the different TGF-β peptides obtained by clostripain digestion is in agreement with the cDNA sequence, except for a few residues which presumably are due to incorrect amino acid assignment in sequencing. The 112 amino acid TGF-β sequence contains 9 cysteines, whereas the rest of the precursor contains only two (positions 187 and 189, Fig. 1b). Previous studies have shown that reduction of the TGF-β dimer of 25 kd results in the generation of two polypeptide chains of 12.5 kd¹⁵. Sequence analysis of the TGF-β amino-terminus and of the TGF-β peptides obtained after clostripain digestion strongly suggest that the TGF-β dimer consists of two identical polypeptides. This homodimeric nature is also supported by the presence of only a single hybridizing DNA fragment upon Southern hybridization of human genomic DNA with a TGF-β exon probe. Chou-Fasman analysis⁵⁰ of the secondary structure shows that the TGF-β polypeptide has an extensive β-sheet character with little, if any, α-helicity. The region immediately preceding the basic dipeptide cleavage site is likely in a α-helical configuration.

A remarkable feature of the TGF-β precursor is that it does not contain a recognizable NH₂-terminal signal peptide sequence typical of most secreted proteins. The mechanism by which TGF-β appears in the extracellular medium is unknown, but might be relatively inefficient since mature TGF-β also can usually be found associated with the rodent or human cells. Nor is it understood how TGF-β is stored inside platelets. Recently cDNA sequences coding for interleukin-1^{51,52} and tumor necrosis factor⁵³ have been determined. Although both appear in the extracellular medium, neither contain a typical NH₂-terminal signal peptide.

One possible mechanism of TGF-β release into the extracellular medium is by cleavage from the precursor anchored in the membrane with the NH₂-terminus in the cytoplasm and the TGF-β sequence on the external side. Most proteins which extend across membranes a single time seem to have a transmembrane region of 20 to 23 uncharged, mostly hydrophobic residues. The single known exception is the T3 subunit of the T-cell receptor⁵⁴ which contains an Asp residue in the transmembrane region. A similar hydrophobic domain cannot be predicted from the deduced amino acid sequence of the TGF-β precursor, making it unlikely that this protein has a single transmembrane domain.

It is conceivable, however, that the TGF-β precursor is a protein with several transmembrane regions. Such proteins, e.g. rhodopsin⁵⁵, the electric eel sodium channel protein⁵⁶ and the major intrinsic protein of the lens gap junctions⁵⁷ have their NH₂-termini in the cytoplasm and contain multiple transmembrane domains which do contain several charged residues. It is possible that some of these charges are neutralized due to the physical proximity of residues with opposite charges, located in neighboring transmembrane regions. In most cases the transmembrane domains are flanked by charged residues, which are often positive on the cytoplasmic side and negative on the extracellular side. Comparison of these features with the TGF-β sequence suggests the TGF-β precursor may belong to this class of membrane proteins, since it contains several potential transmembrane regions and several clusters of charged residues. We propose that the NH₂-terminal 21 amino acids, which are rich in positively charged residues are located on the cytoplasmic side of the first transmembrane region, which is possibly located between residues 22 and 43. This domain is followed at the external side by two Arg residues and a cluster of 5 negatively charged glutamic acid residues (amino acids 54-63). A second transmembrane region could be localized between residues 90 to 114 and precedes a cluster of basic residues (amino acids 119-128) located on the cytoplasmic side. A third membrane spanning region could be found between residues 211-233, following the Arg-Arg dipeptide at position 208. The latter region would contain two positive charges which might neutralize the two negatively charged residues present in the first or second transmembrane region. As a result of this configuration the TGF-β polypeptide and its preceding Arg-Arg residue would be located on the external side of the membrane. This would make cleavage by a dibasic peptidase, localized at that side of the cell membrane^{58,59} possible and would in turn result in the release of TGF-β in the extracellular milieu. This postulated structure of the TGF-β precursor probably implies that, similarly to human rhodopsin⁵⁵ and the major intrinsic protein of the lens fiber membrane⁵⁷, not all potential N-glycosylation sites carry carbohydrate moieties. It should be recognized that the foregoing model is hypothetical and in no way should be construed as limiting the scope of this invention.

For the purposes herein, preTGF-β is defined as the normal TGF-β precursor depicted in Fig. 1 as well as other precursor forms of TGF-β in which the presequence is not that normally associated with TGF-β. These latter forms are to be considered insertional mutants of DNA encoding mature TGF-β. These mutants ordinarily comprise a presequence which is heterologous to TGF-β in the form of a fusion with mature TGF-β. The heterologous presequences preferably are obtained from other secreted proteins, for example pregrowth hormone, preproinsulin, viral envelope proteins, interferons and yeast or bacterial presequences recognized by mammalian host cells. The sequences for these secretory leaders are known, as are suitable sources for DNA encoding same if it is not desired to synthesize the DNA in vitro. They are linked to DNA encoding mature TGF-β by restriction enzyme digestion of the DNA containing the desired signal and the preTGF-β DNA. Synthetic oligonucleotides are prepared in order to introduce unique restriction sites (linkers) and, if necessary, DNA fragments needed to complete any presequence and mature TGF-β coding regions removed during restriction enzyme digestion. The synthesized linkers and/or fragments then are ligated to the restriction enzyme digest fragments containing the substitute signal and TGF-β coding region, inserted into a cloning vector and the vector used to transform bacterial hosts. The mutant presequence thereafter is cloned into an expression vector and used to transform host cells. An illustrative example employing a viral envelope protein presequence is described below.

Optimally, the complete heterologous presequence is linked to the first codon of TGF-β although it is within the scope herein to link the mature TGF-β coding sequence to the complete heterologous presequence plus a short portion, e.g. 21 to 45 base pairs, originating from the DNA encoding the mature heterologous protein. The objective of these constructions is to substitute a high efficiency secretory system for the postulated system of preTGF-β. However, it is by no means necessary to secrete TGF-β in order to produce it in recombinant culture.

Other deletion-insertion mutants include linking mature TGF-β species to viral proteins expressed in large intracellular quantities, e.g. retroviral core proteins, large T antigen from SV40 and the like, or to immunogenic bacterial proteins or polypeptides such as chemotactic polypeptides, in particular the potent chemotactic tripeptide Met-Leu-Phe-.

Expressed mutant preTGF-β, mature TGF-β or fragments thereof will exhibit amino acid sequences that gradually depart from the Fig. 1 sequence as the number and scope of insertions, deletions and substitutions increases. This departure is measured as a reduction in homology between preTGF-β and the mutant. All proteins or polypeptides that display TGF-β anchorage independent growth-promoting biological activity are included within the scope of this invention, regardless of the degree of homology that they show to the Fig. 1 protein. The reason for this is that some regions of preTGF-β, e.g. the presequence, are readily mutated, or even completely deleted as in the case of mature TGF-β, and this biological activity will be retained. On the other hand, deletion of the nine cysteine residues (and accompanying disulfide linkages) in the mature TGF-β molecule will have a substantial adverse impact on this biological activity and in all likelihood would completely abrogate biological activity. In addition, a substitution mutant may exhibit full TGF-β growth-promoting activity and yet be less homologous if residues containing functionally similar amino acid side chains are substituted. Functionally similar refers to dominant characteristics of the side chains such as hydrophobic, basic, neutral or acidic, or the presence or absence of steric bulk. Thus the degree of homology that a given polypeptide bears to preTGF-β is not the principal measure of its identity as TGF-β. However, as a general guide, proteins or polypeptides that share at least some biological activity with mature TGF-β from natural sources and which are substantially homologous with the Fig. 1b sequence, e.g. being about from 40 percent to 100 percent homologous with preTGF-β or any fragment thereof greater than about 20 residues, are to be considered as falling within the scope of the term TGF-β. With respect to neoplastic cell growth inhibiting activity, TGF-β excludes polypeptides known heretofore to exert such growth inhibitory activity, e.g. interferons, tumor necrosis factor and lymphotoxin, but otherwise need not necessarily have homologous regions with the Fig. 1b sequences.

More narrow and specific factors in establishing the identity of a polypeptide as TGF-β are (a) the ability of antisera which are capable of substantially neutralizing the growth inhibitory or the anchorage independent growth promoting activity of mature TGF-β also to substantially neutralize the activity of the polypeptide in question, or the ability of (b) the candidate polypeptide to compete with TGF-β for the TGF-β cell surface receptor. However, it will be recognized that immunological identity and growth promoting identity are not necessarily coextensive. A neutralizing antibody for mature TGF-β of Fig. 1b may not bind a candidate protein because the neutralizing antibody happens to not be directed to a site on TGF-β that is critical for its growth promoting activity. Instead, the antibody may bind an innocuous region and exert its neutralizing effect by steric hinderance. Therefore a candidate protein mutated in this innocuous region might no longer bind the neutralizing antibody, but it would nonetheless be TGF-β in terms of substantial homology and biological activity.

It is important to observe that characteristics such as molecular weight and the like for the native or wild type mature TGF-β of Fig. 1b obtained from placenta or platelets are descriptive only for the native species of TGF-β. The mutants contemplated herein may vary the characteristics of native TGF-β considerably, and this in fact may be the objective of the mutagenesis as is more fully described below. While TGF-β as defined herein includes native TGF-β, other related biologically active polypeptides will fall within the definition. TGF-β species like the insertion mutants, deletion mutants, or fusion proteins described above will bring the mutant outside of the molecular weight established for native TGF-β. For example, fusion proteins with mature TGF-β or TGF-β itself will have a greater molecular weight than native, mature TGF-β, while deletion mutants of mature TGF-β will have a lower molecular weight. Similarly, TGF-β is engineered in order to introduce glycosylation sites, thereby resulting in glycosylated TGF-β, or to substitute serine for cysteine at sites not critical for biological activity. Finally, post-translational processing of human preTGF-β in cell lines derived from nonprimate mammals may produce microheterogeneity in the amino terminal region of mature TGF-β, so that alanine will no longer be the amino terminal amino acid.

Note that the language "capable" of inducing anchorage independent growth in the definition for biological activity means that the preTGF-β or fragments thereof includes polypeptides which can be converted, as by enzymatic polypeptide fragment which exhibits the desired biological activity. Typically, inactive precursors will be fusion proteins in which mature TGF-β is linked by a peptide bond at its carboxyl terminus to an insoluble or gelatinous protein. The sequence at or within the region of this peptide bond is selected so as to be susceptible to proteolytic hydrolysis whereby TGF-β is released, either in vivo for in situ generation or, as part of a manufacturing protocol, in vitro.

While TGF-β ordinarily means human TGF-β, TGF-β from sources such as murine, porcine, equine or bovine is included within the definition of TGF-β so long as it otherwise meets the standards described above for biological activity. TGF-β is not species specific, e.g., murine and human TGF-β are both effective in inducing anchorage independent growth of the same cell line. Therefore, TGF-β from one species can be used in therapy of another species. DNA encoding the TGF-β of other species is obtained by probing cDNA or genomic libraries from such species with labelled human preTGF-β cDNA.

Derivatives of TGF-β are included within the scope of this invention. Derivatives include glycosylated and covalent or aggregatvie conjugates with other TGF-β molecules, dimers or unrelated chemical moieties. Covalent derivatives are prepared by linkage of functionalities to groups which are found in the TGF-β amino acid chains or at the N- or C-termini, by means known in the art. These derivatives may, for example, include: aliphatic or acyl esters or amides of the carboxyl terminus alkylamines or residues containing carboxyl side chains, e.g., conjugates to alkylamines at aspartic acid residues; O-acyl derivatives of hydroxyl group-containing residues and N-acyl derivatives of the amino terminal amino acid or amino-group containing residues, e.g. conjugates with fMet-Leu-Phe or immunogenic proteins and derivatives of the acyl groups are selected from the group of alkyl-moieties (including C3 to C10 normal alkyl), thereby forming alkanoyl species, and carbocylic or heterocyclic compounds, thereby forming aroyl species. The reactive groups preferably are difunctional compounds known per se for use in cross-linking proteins to insoluble matrices through reactive side groups.

Covalent or aggregative derivatives are useful as reagents in immunoassay or for affinity purification procedures. For example, TGF-β is insolubilized by covalent bonding to cyanogen bromide-activated Sepharose by methods known per se or adsorbed to polyolefin surfaces (with or without glutaraldehyde cross-linking) for use in the assay or purification of anti-TGF-β antibodies or cell surface receptors. TGF-β also is labelled with a detectable group, e.g., radioiodinated by the chloramine T procedure, covalently bound to rare earth chelates or conjugated to another fluorescent moiety for use in diagnostic assays, especially for diagnosis of TGF-β levels in biological samples by competitive-type immunoassays.

Mutant TGF-β generally is made by the predetermined, i.e. site specific methods. The objective of mutagenesis is to construct DNA that encodes TGF-β as defined above, i.e., TGF-β which exhibits biological activity.

While the mutation site is predetermined, it is unnecessary that the mutation per se be predetermined. For example, in order to optimize the performance of the mutants at a given position random mutagenesis is conducted at the target codon and the expressed TGF-β mutants screened for optimal activity. Techniques for making substitution mutations at predetermined sites in DNA having a known sequence is well known, for example M13 primer mutagenesis.

TGF-β mutagenesis is conducted by making amino acid insertions, usually on the order of about from 1 to 5 amino acid residues, or deletions of about from 1 to 10 residues. Substitutions, deletions, insertions or any subcombination may be combined to arrive at a final construct. As noted above, insertions include amino or carboxyl-terminal fusions, e.g. with a hydrophobic or immunogenic protein. The mutations in the DNA encoding such mutations should not ultimately place the sequence out of reading frame in an expression vector whereby the resulting protein is not biologically active TGF-β. The mutations also preferably will not create complementary regions that could produce translation-suppressing secondary mRNA structure.

DNA which encodes TGF-β is obtained by chemical synthesis, by screening reverse transcripts of mRNA from placental or other cells or by screening genomic libraries from eukaryotic cells. This DNA need not use the codons set forth in Fig. 1b so long as the host cell recognizes the codons which are used. DNA of this sort is as easily manufactured in vitro as the DNA of Fig. 1b. Also useful herein is nucleic acid, either RNA or DNA, which does not encode TGF-β thereof as defined herein but which nonetheless is capable of hybridizing with such DNA or RNA. Noncoding but hybridizing nucleic acid, while not used in the recombinant synthesis of TGF-β, is useful as an intermediate for making labelled probes in diagnostic assays for TGF-β mRNA or genomic DNA in test cells.

Diagnostic nucleic acid is covalently labelled with a detectable substance such as a fluorescent group, a radioactive atom or a chemiluminescent group by methods known per se. It is then used in conventional Southern or Northern hybridization assays. Such assays are employed in identifying TGF-β vectors and transformants as described in the Examples infra, or for in vitro diagnosis such as detection of TGF-β mRNA in tissues as a measure of mitogenic activity.

TGF-β is synthesized herein in host cells transformed with vectors containing DNA encoding TGF-β. A vector is a replicable nucleic acid construct. Vectors are used either to amplify and/or to express DNA which encodes TGF-β, or to amplify DNA that hybridizes with DNA or RNA encoding TGF-β. An expression vector is a replicable DNA construct in which a DNA sequence encoding TGF-β is operably linked to suitable control sequences capable of effecting the expression of TGF-β in a suitable host. Such control sequences include a transcriptional promoter, an optional operator sequence to control transcription, a sequence encoding suitable mRNA ribosomal binding sites, and sequences which control termination of transcription and translation. For expression of TGF-β in eukaryotic cells the vector also should include DNA encoding a selection gene. However, the selection gene can be supplied by an unlinked plasmid in cotransformation.

Vectors comprise plasmids, viruses (including phage), and integratable DNA fragments i.e., fragments that are integratable into the host genome by recombination. In the present specification, the vector is a plasmid in the sense that it is cloned in bacterial ells, but it integrates into the host cell genome upon cotransformation. However, all other forms of vectors which serve an equivalent function and which are, or become, known in the art are suitable for use herein. Suitable vectors will contain replicon and control sequences which are derived from species compatible with the intended expression host.

DNA regions are operably linked when they are functionally related to each other. For example, DNA for a presequence or secretory leader is operably linked to DNA for a polypeptide if it is expressed as a preprotein which participates in the secretion of the polypeptide; a promoter is operably linked to a coding sequence if it controls the transcription of the sequence; or a ribosome binding site is operably linked to a coding sequence if it is positioned so as to permit translation. Generally, operably linked means contiguous and, in the case of secretory leaders, contiguous and in reading phase.

Cultures of cells derived from multicellular organisms are the preferred host cells herein. In principal, any higher eukaryotic cell culture is workable, whether from vertebrate or invertebrate culture. However, interest has been greatest in vertebrate cells, and propagation of vertebrate cells in culture per se has become a routine procedure in recent years [Tissue Culture, Academic Press, Kruse and Patterson, editors (1973)]. Examples of useful host cell lines are VERO and HeLa cells, Chinese hamster ovary (CHO) cell lines, and WI38, BHK, COS-7 and MDCK cell lines.

Many eukaryotic cells are known to synthesize endogenous TGF-β. Thus, many potential host cells synthesize TGF-β of the host species. This TGF-β therefore is present in the TGF-β produced by transcription and translation of the transforming DNA. For example, hamster TGF-β-transforming CHO cells, and thus is present in cell extracts containing human TGF-β harvested from such cells. While this is not necessarily disadvantageous because animal and human TGF-β is active cross-species, it would be desirable to produce human TGF-β which contains as little of the animal material as possible. This is accomplished by (a) selecting host animal cell lines that synthesize as little of the animal TGF-β as possible, (b) transforming the animal cell line with a vector for high efficiency TGF-β secretion (described above) and recovering human TGF-β from the culture medium or (c) transforming a human cell line, whereby any endogenous hTGF-β will be an advantage rather than a contaminant.

Expression vectors for such cells ordinarily include an origin of replication (for extrachromosomal amplification), a promoter located upstream from the TGF-β coding sequences, along with a ribosome binding site, RNA splice site (if intron-containing TGF-β-encoding genomic DNA is used), a polyadenylation site, and a transcriptional termination sequence.

The transcriptional and translational control sequences in expression vectors to be used in transforming vertebrate cells preferably are provided from viral sources. For example, commonly used promoters are derived from polyoma, Adenovirus 2, and most preferably Simian Virus 40 (SV40). The early and late promoters of SV40 are particularly useful because both are obtained easily from the virus as a fragment which also contains the SV40 viral origin of replication (Fiers et al., 1978, "Nature", 273: 113). Smaller or larger SV40 fragments may also be used, provided the approximately 250 bp sequence extending from the Hind III site toward the Bg1 I site located in the viral origin of replication is included. Further, it is also possible to utilize the human TGF-β genomic promoter, control and/or signal sequences normally associated with TGF-β, provided such control sequences are compatible with and recognized by the host cell.

An origin of replication may be provided either by construction of the vector to include an exogenous origin, such as may be derived from SV40 or other viral (e.g. Polyoma, Adenovirus, VSV, or BPV) source, or may be provided by the host cell chromosomal replication mechanism. If the vector is integrated into the host cell chromosome, the latter is often sufficient.

Rather than using vectors which contain viral origins of replication, one can transform mammalian cells by the method of cotransformation with a selectable marker and the TGF-β DNA. An example of a suitable selectable marker is dihydrofolate reductase (DHFR) or thymidine kinase. Such markers are proteins, generally enzymes that enable the identification of transformant cells, i.e., cells which had been competent to take up exogenous DNA. Generally, identification is by survival of transformants in culture medium that is toxic or from which the cells cannot obtain critical nutrition without having taken up the marker protein. In selecting a preferred host mammalian cell for transfection by vectors which comprise DNA sequences encoding both TGF-β and DHFR, it is appropriate to select the host according to the type of DHFR protein employed. If wild type DHFR protein is employed, it is preferable to select a host cell which is deficient in DHFR thus permitting the use of the DHFR coding sequence as a marker for successful transfection in selective medium which lacks hypoxanthine, glycine, and thymidine. An appropriate host cell in this case is the Chinese hamster ovary (CHO) cell line deficient in DHFR activity, prepared and propagated as described by Urlaub and Chasin, 1980, "Proc. Natl. Acad. Sci." (USA) 77: 4216.

On the other hand, if DNA encoding DHFR protein with low binding affinity for methotrexate (MTX) is used as the controlling sequence, it is not necessary to use DHFR resistant cells. Because the mutant DHFR is resistant to MTX, MTX containing media can be used as a means of selection provided that the host cells are themselves MTX sensitive. Most eukaryotic cells which are capable of absorbing MTX appear to be methotrexate sensitive. One such useful cell line is a CHO line, CHO-K1 (ATCC No. CCL 61).

Other methods suitable for adaptation to the synthesis of TGF-β in recombinant vertebrate cell culture are described in M-J. Gething et al., "Nature" 293:620-625 (1981); N. Mantei et al., "Nature" 281:40-46; A. Levinson et al., EP 117,060A and 117,058A.

TGF-β is recovered from lysed, transformed cells and soluble cell debris separated by centrifugation. Alternatively, the culture supernatants from transformed cells that secrete TGF-β are simply separated from the cells by centrifugation. Then the TGF-β generally is purified by methods known in the art^{15,16,17} using gel filtration in the presence of acid followed by HPLC and elution on an acetonitrile gradient. Such methods are not necessarily coextensive with the purification required for a therapeutic product.

As a further or substitute purification step, cell lysates or supernatants are heated for a period and at a temperature sufficient to denature and precipitate contaminant proteins but not TGF-β; TGF-β is a remarkably heat stable protein, perhaps as a result of extensive disulfide bond formation. As a result, the heating should be conducted in a medium that contains low amounts of disulfide reagents such as dithiothreitol or the like. Heating also is combined with acidifcation since TGF-β is known to be stable to 1M acetic acid.

Mature, native TGF-β is not glycosylated. Therefore it is separate from any residual contaminant heat- and acid-stable glycoproteins by adsorbing the glycoproteins on lectin columns such as lentil lectin-linked sepharose. This step, less desirably, can go before the heat and acid treatment. TGF-β will elute with the unadsorbed fraction.

If high purity product is desired the crude or partially purified mixture thereafter is subjected to chromotofocusing.

TGF-β is prepared for administration by mixing TGF-β at the desired degree of purity with physiologically acceptable carriers, i.e., carriers which are nontoxic to recipients at the dosages and concentrations employed. Ordinarily, this will entail combining TGF-β with buffers, antioxidants such as ascorbic acid, low molecular weight (less than about 10 residues) polypeptides, proteins, amino acids, carbohydrates including glucose or dextrins, chelating agents such as EDTA, and other excipients. TGF-β for use in therapeutic administration must be sterile. This is readily accomplished by filtration through sterile filtration (0.2 micron) membranes. TGF-β ordinarily will be stored as an aqueous solution since it is highly stable to thermal and oxidative denaturation.

Two types of application of TGF-β compositions have been contemplated.

The first, and preferred, application is topically for the promotion of surface wound healing. There are no limitations as to the type of wound or other traumata that can be treated, and these include (but are not limited to): first, second and third degree burns (especially second and third degree); epidermal and internal surgical incisions, including those of cosmetic surgery; wounds, including lacerations, incisions, and penetrations; and surface ulcers including decubital (bed-sores), diabetic, dental, hemophiliac, and varicose.

When TGF-β compositions are applied to burns in the form of a sterile irrigant, preferably in combination with a physiological saline solution, or in the form of ointments or suspensions, preferably in combination with purified collagen. The compositions also may be impregnated into transdermal patches, plasters, and bandages, preferably in a liquid or semi-liquid form. Automicrobial agents such as silver sulfadiazine should be included in such articles or compositions. Debridement agents such as proteolytic enzymes also can be included if they do not hydrolyze TGF-β or a hydrolysis-resistant TGF-β mutant is employed.

The second application is systemic administration for the healing of internal wounds and similar traumata. Such an application is useful provided that there are no, or limited, undesirable side-effects, such as the stimulation of neoplastic cellular growth in patients with cancer. TGF-β commpositions for systemic administration preferably are formulated as sterile, isotonic parenteral injections or infusions.

TGF-β optionally is combined with activating agents such as TGF-α, EGF or other growth factors. The amount of activating agent present depends directly upon the amount of TGF-β present in the activated compositions as administered to the recipient.

The amount of activated composition to be used will vary depending upon the growth factors selected and the clinical status of the patient.

However, it can generally be stated that TGF-β should preferably be present in an amount of at least about 1.0 nanogram per milliliter of combined composition, more preferably in an amount up to about 1.0 milligram per milliliter. Since TGF-β compositions both provoke and sustain cellular regeneration, a continual application or periodic reapplication of the compositions is indicated.

In order to simplify the Examples certain frequently occurring methods will be referenced by shorthand phrases.

Plasmids are designated by a low case p preceded and/or followed by capital letters and/or numbers. The starting plasmids herein are commercially available, are publicly available on an unrestricted basis, or can be constructed from such available plasmids in accord with published procedures. In addition, other equivalent plasmids are known in the art and will be apparent to the ordinary artisan.

"Digestion" of DNA refers to catalytic cleavage of the DNA with an enzyme that acts only at certain locations in the DNA. Such enzymes are called restriction enzymes, and the sites for which each is specific is called a restriction site. The various restriction enzymes used herein are commercially available and their reaction conditions, cofactors and other requirements as established by the enzyme suppliers were used. Restriction enzymes commonly are designated by abbreviations composed of a capital letter followed by other letters representing the microorganism from which each restriction enzyme originally was obtained and then a number designating the particular enzyme. In general, about 1 µg of plasmid or DNA fragment is used with about 2 units of enzyme in about 20 µl of buffer solution. Appropriate buffers and substrate amounts for particular restriction enzymes are specified by the manufacturer. Incubation times of about 1 hour at 37°C are ordinarily used, but may vary in accordance with the supplier's instructions. After incubation, protein is removed by extraction with phenol and chloroform, and the digested nucleic acid is recovered from the aqueous fraction by precipitation with ethanol. Digestion with a restriction enzyme infrequently is followed with bacterial alkaline phosphatase hydrolysis of the terminal 5' phosphates to prevent the two restriction cleaved ends of a DNA fragment from "circularizing" or forming a closed loop that would impede insertion of another DNA fragment at the restriction site. Unless otherwise stated, digestion of plasmids is not followed by 5' terminal dephosphorylation. Procedures and reagents for dephosphorylation are conventional (T. Maniatis et al., 1982, Molecular Cloning pp. 133-134).

"Recovery" or "isolation" of a given fragment of DNA from a restriction digest means separation of the digest on polyacrylamide or agarose gel by electrophoresis, identification of the fragment of interest by comparison of its mobility versus that of marker DNA fragments of known molecular weight, removal of the gel section containing the desired fragment, and separation of the gel from DNA. This procedure is known generally. For example, see R. Lawn et al., 1981, "Nucleic Acids Res."9: 6103-6114, and D. Goeddel et al., 1980, "Nucleic Acids Res." 8: 4057.

"Southern Analysis" is a method by which the presence of DNA sequences in a digest or DNA-containing composition is confirmed by hybridization to a known, labelled oligonucleotide or DNA fragment. For the purposes herein, unless otherwise provided, Southern analysis shall mean separation of digests on 1 percent agarose, denaturation and transfer to nitrocellulose by the method of E. Southern, 1975, "J. Mol. Biol." 98: 503-517, and hybridization as described by T. Maniatis et al., 1978, "Cell" 15: 687-701.

"Transformation" means introducing DNA into an organism so that the DNA is replicable, either as an extrachromosomal element or chromosomal integrant. Unless otherwise provided, the method used herein for transformation of E. coli is the CaCl₂ method of Mandel et al., 1970, "J. Mol. Biol." 53: 154.

"Ligation" refers to the process of forming phosphodiester bonds between two double stranded nucleic acid fragments (T. Maniatis et al., Id., p. 146). Unless otherwise provided, ligation may be accomplished using known buffers and conditions with 10 units of T4 DNA ligase ("ligase") per 0.5 µg of approximately equimolar amounts of the DNA fragments to be ligated.

"Preparation" of DNA from transformants means isolating plasmid DNA from microbial culture. Unless otherwise provided, the alkaline/SDS method of Maniatis et al., Id. p. 90., may be used.

"Oligonucleotides" are short length single or double stranded polydeoxynucleotides which are chemically synthesized by known methods and then purified on polyacrylamide gels.

All literature citations are expressly incorporated by reference.

### Example 1

### Purification and Sequence Analysis of Human TGF-β

The known purification method of Assoian et al.¹⁵ was scaled up and modified to obtain enough homogeneously pure human TGF-β for amino acid sequencing. 250 units of human platelets were extracted in a Waring blender with 1 l of acid-ethanol. Addition of 4 l of ether gave rise to a precipitate which was collected by vacuum filtration over Whatman No. 1 paper. The precipitate was dissolved overnight in 50 ml of 1M acetic acid and purified by gel filtration on a Biogel P-60 column (10x100 cm), equilibrated in 1M acetic acid. The fractions containing TGF-β were identified by analytical SDS-polyacrylamide gel electrophoresis and bioassay¹⁵. Peak fractions were pooled, freeze-dried and redissolved in 20 ml 1M acetic acid, 8M urea. Subsequent gel filtration over a Biogel P-60 column (5x90 cm) in 1M acetic acid, 8M urea yielded about 50 percent pure TGF-β. These peak fractions were then diluted with 1 volume of water and applied to a semipreparative RPP C18 (Synchropak) HPLC column in 0.1 percent trifluoroacetic acid and eluted with a 20-50 percent acetonitrile gradient. The TGF-β thus obtained was quantitated by amino acid analysis, showing a yield of about 0.5 mg per preparation. Denaturing SDS-polyacrylamide gel electrophoresis was performed as described⁶⁰. In agreement with previous work the non-reduced TGF-β migrated as a 25 kD protein in a SDS-polyacrylamide gel, while reduction with β-mercaptoethanol converted it into a 12.5 kD species. This suggested that TGF-β consists of two 12.5 kd polypeptide chains linked by intermolecular disulfide bridges¹⁵.

In order to obtain protein sequence information, the purified TGF-β was reduced, alkylated and subjected to amino-terminal sequence analysis. 1.2 nmole of TGF-β was dialyzed into 8M urea and reduced by incubation in 0.1M Tris-HCl (pH 8.5), 10 mM dithiothreitol, 8M urea. Subsequent alkylation took place in the presence of 50 mM iodoacetate at room temperature in the dark. This reaction was terminated after 30 min. by addition of an excess β-mercaptoethanol and dialysis. 0.7 nmole of this TGF-β was used for the direct NH₂-terminal sequence analysis. 1.2 nmole of reduced and alkylated TGF-β was digested in 0.75 M urea, 50 mM NH₄HCO₃, 5mM dithiothreitol for 24 hours with 1 percent clostripain¹⁵. An additional 1 percent of clostripain was added after 12 hours reaction time. The reaction products were separated on a Synchropak RPP C18 reverse phase column (4.6 x 250 mm) with a 0-70 percent acetonitril gradient in 0.1 percent trifluoroacetic acid. Sequence determination took place using either an extensively modified Beckman 890C spinning cup sequencer⁶¹ or a vapor phase sequencer as described by Hewick et al.⁶² (Applied Biosystems, model 470A), with amino acid derivative identification by reversed phase HPLC on a Rainin Microsorb C-8 column. The amino acid sequence of several peptides was determined. One of these fragments was the NH₂-terminal segment, while another large peptide yielded a 37 amino acid sequence which overlapped the NH₂-terminal sequence and established 60 residues of contiguous sequence.

Unmodified TGF-β was also treated with CNBr. Cleavage at the methionine residue resulted in the complete loss of biological activity, documenting that at least part of this C-terminal octapeptide is needed for biological activity (data not shown).

### Example 2

### Isolation of a TGF-β Exon

The approach we followed for the identification of the nucleotide sequence encoding TGF-β was similar to the strategy used previously for TGF-α⁷. Long oligonucleotides designed on the basis of the partial protein sequence were used as hybridization probes for the identification of a TGF-β exon in a human genomic DNA library. The TGF-β exon was then used as a probe for the isolation of TGF-β cDNAs.

Two 44-base-long deoxyoligonucleotides, βLP1 and βLP2, complementary to sequences coding for amino acids 3 to 17 and 30 to 44, respectively, were chemically synthesized^{63,64}. The choice of nucleotide sequence was based upon the codon bias observed in human mRNAs²⁶. CpG dinucleotides, which are relatively rare in vertebrate DNA²⁷, were avoided whenever possible. In addition, sixteen 14-mers were synthesized which are complementary to all possible codons for amino acids 13 to 17. These deoxyoligonucleotides and the corresponding amino acid sequence are shown below. 
NH2-AlaLeuAspThrAsnTyrCysPheSerSerThrGluLysAsnCysCysValArgGluLeuTyr
.. .. .. .. .. ..      .. .. ..    .. .. .. ..
CTGTGGTTGATAACGAAGAGGAGGTGTCTCTTCTTGACGACGCA 5'

  C  G  G  G
TT TT AC AC CA
  T  A  A  A

LeuGlyTrpLysTrpIleHisGluProLysGlyTyrHisAlaAsnPheCysLeuGlyProCysProTyr
..... ..... .. .. .. .. .. .. .. .. .. .. .. ..
ACCTTCACCTAGGTACTCGGTTTCCCGATAGTACGGTTGAAGAC 5'
The nucleotides marked with a dot are residues for which there is no ambiguity in the codon.

A human genomic DNA library²⁸ was screened under low stringency hybridization conditions using ³²P-labelled βLP-1 as probe. Approximately 7.5 x 10⁵ recombinant phage from a human genomic fetal liver library²⁸ were hybridized using low stringency conditions⁶⁵ with the ³²P-labelled 44-mer βLP-1 after replica plating onto nitrocellulose filters⁶⁶. DNA was prepared from 58 of the hybridizing phage and hybridized with the ³²P-labelled βLP-1 and βLP-2 oligonucleotides using the "dot blot" analysis method⁶⁷ and Southern hybridization⁶⁸ of BamHI digestion mixtures. The two phage DNAs which hybridized with both oligonucleotides were digested and probed with the pool of ³²P-labelled 14-mers again by Southern hybridization. 14-mer hybridizations were performed at 37°C in 6xSSC, 0.5 percent NP40, 6mM EDTA, 1X Denhart's solution and 50 µg/ml salmon sperm DNA. Several washes were performed at room temperature in 6xSSC before autoradiography. DNA from phage βλ58 hybridized with the oligonucleotides βLP-1, βLP-2 and with the 14-mer pool. The sequences hybridizing to βLP-2 and the 14-mers were localized within the same 4.2 kbp BamHI fragment, while probe βLP-1 hybridized to a 20 kb BamHI fragment. The hybridizing BamHI fragments were subcloned into pBR322. The nucleotide sequence of smaller hybridizing fragments was determined by dideoxynucleotide chain termination method⁶⁹ after subcloning into M13 derivatives⁷⁰.

The screening of the genomic DNA library resulted in the isolation of an exon coding only part of the TGF-β coding sequence (residues 10 to 60, Fig. 3). In order to obtain the entire TGF-β coding sequence, this exon was used as a probe to screen a λgt10 based cDNA library derived from human term placenta mRNA.

### Example 3

### Isolation of TGF-β cDNAs

Total RNA was extracted⁷¹ from the different cell sources and the polyadenylated mRNA fraction was isolated by oligo(dT)-cellulose chromatography⁷². The cDNA was prepared⁷³ by priming with dT₁₂₋₁₈ or the deoxyoligonucleotide ACACGGGTTCAGGTAC (complementary to nucleotides 1270 to 1286). The double-stranded cDNA was treated with nuclease S1 (Miles Laboratories) followed by E. coli DNA polymerase I Klenow fragment (Boehringer Mannheim) and subcloned into EcoRI cleaved λgt10 as described⁷⁴, except that asymmetric EcoRI linkers⁷⁵ were used, thus avoiding the need for the EcoRI methylase treatment. The recombinant phage were plated on E. coli C600 Hfl⁷⁴ and replica plated onto nitrocellulose filters⁶⁶. These were hybridized with ³²P-labelled⁷⁶ specific restriction fragments at 42°C in 50 percent formamide, 5x SSC, 50 mM sodium phosphate pH 6.8, 0.1 percent sodium pyrophosphate, 5x Denhardt's solution, 50 µg/ml salmon sperm DNA and washed in 0.2x SSC, 0.1 percent SDS at the same temperature. Low stringency hybridization conditions⁶⁵ were used in the case of the ³²P-labelled deoxyoligonucleotides. The nucleotide sequence of the TGF-β cDNA restriction fragments was determined by the dideoxyoligonucleotide chain termination method⁶⁹ after subcloning into M13 phage derivatives⁷⁰. The cDNAs obtained are schematically shown in Fig. 1a. λβC1 was isolated from a human placenta cDNA library using the genomic exon Fig. 3) as probe. The screening of approximately 750,000 oligo-dT primed placenta cDNA clones resulted in the isolation of one TGF-β cDNA (λβC1) of about 1,050 bp. The previously determined partial TGF-β sequence established the reading frame and revealed the sequence coding for the complete TGF-β polypeptide. This sequence begins with the NH₂-terminal alanine residue and is followed 112 codons later by a stop codon, only 20 base pairs from the 3' end. The λβC1 EcoRI cDNA insert was used in turn to screen the A172 glioblastoma cDNA library leading to the isolation of λβC3.19. Screening of a specifically primed HT1080 fibrosarcoma cDNA library with the ³²P-labelled KpnI-KpnI and the upstream EcoRI-KpnI fragment of the λβC3.19 cDNA insert yielded λβC4.10, 4.33 and 4.37. Another similar library was screened with the λβC4.33 insert and a synthetic 40-mer corresponding to necleotides 1-40, leading to the isolation of λβC5.7b.

None of more than seventy TGF-β cDNAs isolated from different oligo(dT)-primed cDNA libraries contained more than a few nucleotides of 3' untranslated regions, the 3' untranslated sequence was determined using cloned genomic DNA. Hybridization analysis showed that the 3' end of the λβC1 cDNA insert was present in the genomic DNA phage βλ58. DNA sequence analysis revealed the presence of an exon coding for the carboxy terminal part of TGF-β, followed by the stop codon and the 3' untranslated end (Fig. 1b). An AATAAA hexanucleotide sequence³² was encountered 500 bp downstream from the termination codon, thus permitting an assignment of the putative polyadenylation site. Assuming this is indeed the polyadenylation signal, the calculated size of TGF-β mRNA is in close agreement with the 2.3 to 2.5 kb length determined from the Northern hybridization experiments (Example 4). Additional screening of oligo(dT)-primed placenta and HT1080 cDNA libraries using the genomic DNA probe for the 3' untranslated end did not identify a single hybridizing cDNA phage.

### Example 4

### Diagnostic Method Using TGF-β cDNA Probes

Polyadenylated RNA was recovered from the hepatoma HEP-G2, Wilms tumor TuWi, glioblastoma A172, bladder carcinoma T24, squamous epidermoid carcinoma A431, mammary carcinoma MCF-7, nasopharyngeal carcinoma KB, fibrosarcoma HT1080, Burkitt lymphoma B-lymphoblasts Daudi and Raji, T-lymphoblast Molt-4. Peripheral blood lymphocytes were prepared and mitogen-induced with staphylococcal enterotoxin B and phorbol myristate as described⁵³. RNA was harvested in this case after 24 hours. 4 µg of polyadenylated mRNA was electrophoresed into formaldehyde-1.2 percent agarose gel²⁹ and blotted onto nitrocellulose filters³⁰. The ³²P-labelled⁷⁶ EcoRI cDNA insert of λβC1 was used as probe under high stringency conditions used above. Comparison with the position of the 28S and 18S rRNA on the gel suggests a length of 2.3-2.5 kb for the TGF-β mRNA. In some cases a smaller mRNA species may be present, although partial degradation of the mRNA cannot be excluded.

TGF-β mRNA was detectable in all human tumor cell lines including tumor cells of neuroectodermal origin, such as TuWi (Wilms Tumor) and A172 (glioblastoma), and the carcinoma cell lines T24 bladder carcinoma, A431 (squamous epidermoid carcinoma), MCF-7 (mammary carcinoma) and KB (nasopharyngeal carcinoma). HT1080, a fibrosarcoma derived cell line, which we had chosen as a source of mRNA for the cDNA cloning, contained relatively high levels of TGF-β mRNA. TGF-β mRNA was not only present in cell lines derived from solid tumors of meso-, endo- and ectoblastic origin, but was also detectable in tumor cell lines of hematopoietic origin, e.g. Daudi (Burkitt lymphoma B-lymphoblast), Raji (Burkitt lymphoma B-lymphoblast), and Molt-4 (T-cell leukemia). The presence of TGF-β mRNA is not restricted to tumor cells, since it is clearly detectable in placenta and peripheral blood lymphocyte (PBL) mRNA. Strikingly, the level of TGF-β mRNA is significantly elevated after mitogenic stimulation of PBLs. TGF-β mRNA was not detectable in human liver, yet was present in the HEP-G2 hepatoma cell line. In all cases, the TGF-β mRNA migrated as a species of an apparent length of 2.3 to 2.5 kbases. In some cases a smaller mRNA species of about 1.8 to 1.9 kb may be present, although this could be due to partial degradation of the mRNA.

### Example 5

### Recombinant Synthesis of TGF-β

The plasmid used for recombinant synthesis of TGF-β was pMBTE6. Following is a prophetic method for making this plasmid that is a variant method preferred over the more complex method actually employed in its construction.

p342E⁷⁹ is digested with EcoRI, blunted with E. coli DNA polymerase I (Klenow fragment) and the four dNTPs, digested with SalI and Fragment 1 (containing the Amp^{r} gene of pBR322) recovered.

p342E is simultaneously digested with SalI and HindIII and the HBsAg-encoding fragment is recovered as Fragment 2.

Finally, the SV40 genome is simultaneously digested with HindIII and HincII, and the 596 bp fragment containing the SV40 origin and early promoter recovered as Fragment 3.

Fragments 1, 2 and 3 are ligated in a three way ligation and the ligation mixture transformed into E. coli strain 294 (ATCC 31446). The transformed culture is plated on ampicillin media plates and resistant colonies selected. p342E-blunt was recovered from a transformant colony.

p342E blunt is digested simultaneously with HindIII and EcoRI and the large vector fragment recovered. This fragment is ligated to a polylinker having the following sequence 
EcoRI
A A G C T T A T C G A T T C T A G A A T T C
HindIII ClaI XbaI
and the ligation mixture used to transform E. coli ATCC 31446 as described above. pCVSV-HBs is recovered from an ampicillin-resistant transformant.

pCVSV-HBs is digested with Hind III and EcoRI simultaneously and the vector fragment isolated (the 18 bp HindIII-EcoRI fragment will not appear in the gel due to its small size).

pgD-DHFR-Trunc (EP-A-0139417), a plasmid containing DNA encoding the herpes simplex gD protein, is simultaneously digested with StuI and HindIII and the approximately 760 bp fragment recovered which contains DNA encoding the herpes simplex signal peptide and the coding region for the N-terminal part of the mature HSV-1gD protein. Plasmid pJ2.9 from EP-A-0139417 can be used in the same fashion.

pβC1 (Fig. 1a) is digested with SmaI and BamHI, and the 480 bp fragment recovered. This fragment contains most of the sequence coding for preTGF-β including the sequence coding for the N-terminus of mature TGF-β through residue 274.

pβC1 is digested with BamHI and EcoRI and the 270 bp fragment recovered. These two separate digestions of pβC1 aliquots were conducted because the BamHI-EcoRI pβC1 fragment contains a SmaI site. The 270 bp fragment contains the sequence coding for the rest of the TGF-β molecule and extends 20 bp beyond the stop codon.

The pCVSV-HBs vector fragment is ligated in a four way ligation with the foregoing 760, 270 and 480 bp fragments. The resulting construction (pCVSVgD) thus contained a hybrid coding sequence (Herpes simplex gD-1 signal peptide and part of the gD-1 envelope protein linked in frame to the the preTGF-β precursor fragment) under the control of the SV40 early promoter. This hybrid coding sequence is in turn followed by the 3' untranslated sequence and the polyadenylation signal of the hepatitis surface antigen.

pCVSVgD is digested with EcoRI, blunted with Klenow and the four dTNPs, and thereafter digested with PstI. Two fragments are so obtained, with the fragment including the hybrid coding sequence and the SV40 promoter (fragment A) being recovered.

pCVSVgD is digested with BamHI, blunted with Klenow and the four dTNPs, and thereafter digested with PstI. Four fragments are obtained after these digestions. The fragment containing the pBR322 origin and Amp^{r} gene (about 1900 bp) is recovered as Fragment B.

Fragments A and B are ligated and the ligation mixture used to transform E. coli ATCC 31,446. Plasmid pMBTE6 is recovered from an Amp^{r} colony.

Plasmid pMBTE6 was transfected into DHFR deficient CHO cells (Urlaub and Chasin, 1980, P.N.A.S. 77, 4216-4220) together with the plasmid pFD11 (Simonsen and Levinson, 1983, P.N.A.S. 80, 2495-2499). The latter plasmid encodes DHFR, thereby conferring methotrexate resistance on the transfected cells and allowing for selection of TGF-β expressing transformants. Any DHFR⁻ mammalian host cell is suitable for use. Alternatively, one can use any mammalian host cell, cotransform the host cell with a plasmid encoding neomycin resistance, and identify transformants by their ability to grow in neomycin-containing medium.

The transfected CHO cells were selected by culturing in HGT⁻ medium. The cells were allowed to grow to confluency in 15 cm diameter plates. The cells thereafter were cultured in serum-free medium for 48 hrs prior to harvest. The culture medium was decanted from the plates and assayed in a soft agar assay for the presence of TGF-β as described⁷⁸.

50 ml supernatant medium was lyophilized and dissolved in 700 µl 4mM HCl-0.1 percent bovine serum albumin. 200 µl of this solution and of serial three-fold dilutions were assayed. The number of colonies in soft agar with a diameter of >89 µm were counted. The maximal response (plateau-value) obtained in the presence of saturating levels of TGF-β was about 1500 per plate. Less than 50 colonies were obtained in the absence of TGF-β. A half maximal response was obtained with a 9-fold dilution of the sample derived from the cells transformed with a negative control plasmid. Calculations from the values obtained by serial dilution of the MBTE6 supernatant showed that the half maximal value was obtained at a 70 fold dilution.

The assays of serial dilutions show that cells transformed with MBTE6 and pFD11 synthesize about 8-10 times more TGF-β per ml of medium than do CHO cells transfected with pFD11 alone or with pFD11 together with a control plasmid (one which was similar to pMBTE6 except that the Herpes coding sequence is replaced by the sequence coding for the bacterial STII signal peptide), even prior to subcloning and selection in MTX-containing media. This additional amount of TGF-β is human TGF-β.

Since biologically active TGF-β is found in the culture medium it is concluded that CHO cells cleave preTGF-β in the same fashion as do human cells in vivo to secrete mature mature TGF-β. This conclusion is very much strengthened by the fact that the slope of the TGF-β concentration dilution curve in the soft agar is identical for both the endogenous natural TGF-β and the recombinant TGF-β, thus reflecting a similar if not identical affinity for the TGF-β receptor.

### Bibliography

1. De Larco, J.E. and Todaro, G.J. Proc. Natl. Acad. Sci. USA 75, 4001-4005 (1978).
2. Roberts, A.B., Frolik, C.A., Anzano, M.A. and Sporn, M.B. Fed. Proc. 42, 2621-2625 (1983).
3. Todaro, G.J., Fryling, C. and De Larco, J.E. Proc. Natl. Acad. Sci. USA 77, 5258-5262 (1980).
4. Marquardt, H., Hunkapiller, M.W., Hood, L.E. and Todaro, G.J. Science 223, 1079-1082 (1984).
5. Roberts, A.B., Lamb, L.C., Newton, D.L., Sporn, M.B., De Larco, J.E. and Todaro, G.J. Proc. Natl. Acad. Sci. USA 77, 3494-3498 (1980).
6. Ozanne, B., Fulton, R.J. and Kaplan, P.L. J. Cell Physiol. 105, 163-180 (1980).
7. Derynck, R., Roberts, A.B., Winkler, M.E., Chen, E.Y. and Goeddel, D.V. Cell 38, 287-297 (1984).
8. Lee, D.C., Rose, R.M., Webb, N.R. and Todaro, G.J. Nature 313, 489-491 (1985).
9. Linsley, P.S., Hargreaves, W.R., Twardzik, D.R. and Todaro, G.J. Proc. Natl. Acad. Sci. USA 82, 356-360 (1985).
10. Roberts, A.B., Anzano, M.A., Lamb, L.C., Smith, J.M. and Sporn, M.B. Proc. Natl. Acad. Sci. USA 78, 5339-5343 (1981).
11. Roberts, A.B., Anzano, M.A., Lamb, L.C., Smith, J.M., Frolik, C.A., Marquardt, H., Todaro, G.J. and Sporn, M.B. Nature 295, 417-419 (1982).
12. Roberts, A.B., Anzano, M.A., Meyers, C.A., Wideman, J., Blacher, R., Pan, Y.C., Stein, S., Lehrman, S.R., Smith, J.M., Lamb, L.C., and Sporn, M.B. Biochemistry 22, 5692-5698 (1983).
13. Frolik, C.A., Dart, L.L., Meyers, C.A., Smith, D.M. and Sporn, M.B. Proc. Natl. Acad. Sci. USA 80, 3676-3680 (1983).
14. Childs, C.B., Proper, J.A., Tucker, R.F. and Moses, H.L. Proc. Natl. Acad. Sci. USA 79, 5312-5316 (1982).
15. Assoian, R.K., Komoriya, A., Meyers, C.A., Miller, D.M. and Sporn, M.B. J. Biol. Chem. 258, 7155-7160 (1983).
16. Assoian, R.K., Grotendorst, G.R., Miller, D.M. and Sporn, M.B. Nature 309, 804-806 (1984).
17. Sporn, M.B., Roberts, A.B., Shull, J.H., Smith, J.M., Ward, J.M. and Sodek, J. Science 219, 1329-1331 (1983).
18. Frolik, C.A., Wakefield, L.M., Smith, D.M. and Sporn, M.B. J. Biol. Chem. 259, 10995-11000 (1984).
19. Tucker, R.F., Branum, E.L., Shipley, G.D., Ryan, R.J. and Moses, H.L. Proc. Natl. Acad. Sci. USA 81, 6757-6761 (1984).
20. Assoian, R.K., Frolik, C.A., Roberts, A.B., Miller, D.M. and Sporn, M.B. Cell 36, 35-41 (1984).
21. Tucker, R.F., Volkenant, M.E., Branum, E.L. and Moses, H.L. Cancer Res. 43, 1581-1586 (1983).
22. Anzano, M.A., Roberts, A.B., De Larco, J.E., Wakefield, L.M., Assoian, R.K., Roche, N.S., Smith, J.E., Lazarus, J.E. and Sporn, M.B. Molec. Cell. Biology 5, 242-247 (1985).
23. Roberts, A.B., Anzano, M.A., Wakefield, L.A., Roche, N.S., Stern, D.F. and Sporn, M.D. Proc. Natl. Acad. Sci. USA 82, 119-123 (1985).
24. Tucker, R.F., Shipley, G.D., Moses, H.L. and Holley, R.W. Science 226, 705-707 (1984).
25. Mitchell, W.M. Meth. Enzymol. XLVII, p. 165-170 (1977).
26. Grantham, R., Gautier, C., Gouy, M., Jacobzone, M. and Mercier, R. Nucl. Acids Res. 9, 43-73 (1981).
27. Bird, A.P. Nucl. Acids Res. 8, 1499-1504 (1980).
28. Lawn, R.M., Fritsch, E.F., Parker, R.C., Blake, G. and Maniatis, T. Cell 15, 1157-1174 (1978).
29. Dobner, P.R., Kawasaki, E.S., Yu, L.Y. and Bancroft, F.C. Proc. Natl. Acad. Sci. USA 78, 2230-2234 (1981).
30. Thomas, P.S. Proc. Natl. Acad. Sci. USA 77, 5201-5205 (1980).
31. Volckaert, G., Tavernier, J., Derynck, R., Devos, R. and Fiers, W. Gene 15, 215-223 (1981).
32. Proudfoot, N.J. and Brownlee, G.G. Nature 253, 211-214 (1976).
33. Levy, W.P., Rubinstein, M., Shively, J., Del Valle, U., Lai, C.-Y., Moschera, J., Brink, L., Gerber, L., Stein, S. and Pestka, S. Proc. Natl. Acad. Sci. USA 78, 6186-6190 (1981).
34. Rinderknecht, E., O'Connor, B.H. and Rodriguez, H. J. Biol. Chem. 259. 6790-6797 (1984).
35. Kozak, M. Nucl. Acids Res. 12, 857-872 (1984).
36. Shepherd, J.C.W. Proc. Natl. Acad. Sci. USA 78, 1596-1600 (1981).
37. Battey, J., Moulding, C., Taub, R., Murphy, W., Stewart, T., Potter, H., Lenoir, G. and Leder, P. Cell 34, 779-787 (1983).
38. No citation.
39. No citation.
40. McKnight, S.L., Kingsbury, R.C., Spence, A. and Smith, M. Cell 37, 253-262 (1984).
41. Dynan, W.S. and Tjian, R. Cell 35, 79-87 (1983).
42. Gidoni, D., Dynan, W.S. and Tjian, R. Nature 312, 409-413 (1984).
43. Benoist, C., O'Hare, K., Braetnach, R. and Chambon, P. Nucl. Acids Res. 127-142 (1980).
44. Noda, M., Furutani, Y., Takahashi, H., Toysato, M., Hirose, T., Imayama, S., Nakanishi, S. and Numa, S. Nature 295, 202-206 (1982).
45. Gubler, U., Seeburg, P., Hoffman, B.J., Gage, L.P. and Udenfriend, S. Nature 295, 206-208 (1982).
46. Amara, S.G., Jonas, V., Rosenfeld, M., Ong, E.S. and Evans, R.M. Nature 298, 240-244 (1982).
47. Nakanishi, S., Inoue, A., Nakamura, M., Chang, A.C.Y., Cohen, S.N. and Numa, S. Nature 278, 423-427 (1979).
48. Kyte, J. and Doolittle, R.F. J. Mol. Biol. 157, 105-132 (1982).
49. Winzler, R.J. in Hormonal Proteins and Peptides 1, (Li, C.I., ed.) (New York, Academic Press) p. 1-15 (1973).
50. Garnier, J., Osguthorpe, D.J. and Robson, B. J. Mol. Biol. 120, 97-120 (1978).
51. Arnon, P.E., Webb, A.C., Rosenwasser, L.J., Mucci, S.F., Rich, A., Wolff, S.M. and Dinarello, C.A. Proc. Natl. Acad. Sci. USA 81, 7907-7911 (1984).
52. Lomedico, P., Guebler, U., Hellman, P., Dukovich, M., Giri, J.G., Pan, Y.-C., Collier, K., Semionow, R., Chua, A.D. and Mizel S.B. Nature 312, 458-462 (1984).
53. Pennica, D., Nedwin, G., Hayflick, J.S., Seeburg, P.H., Derynck, R., Palladino, M.A., Kohr, W.J., Aggarwal, B.B. and Goeddel, D.V. Nature 312, 724-729 (1984).
54. Van den Elsen, Shepley, B.-A., Borst, J., Coligan, J.E., Markham, A., Orkin, S. and Terhorst, C. Nature 312, 413-418 (1984).
55. Nathans, J. and Hogness, D. Proc. Natl. Acad. Sci. USA 4851-4855 (1984).
56. Noda, M., Shimizu, S., Tanabe, T., Takai, T., Kayano, T., Ikeda, T., Takahashi, H., Nakayama, H., Kanaoka, Y., Minamino, N., Kangawa, K., Matsuo, H., Raftery, M.A., Hirose, T., Inayama, S., Hayashida, H., Miyata, T. and Numa, S. Nature 312, 121-127 (1984).
57. Gorin, M.B., Yancey, B., Cline, J., Revel, J.-P. and Horwitz, J. Cell 39, 49-59 (1984).
58. Noe, B.D., Moran, M.N. and Spiess, J. in Peptides: Structure and Function (Pierce Chemical Company; Hurby, V. and Rich, D.H., eds.). p. 219-228 (1983).
59. Devault, A., Zollinger, M. and Crine, P. J. Biol. Chem. 259, 5146-5151 (1984).
60. Laemmli, U.K. Nature 227, 680-685 (1970).
61. Rodriguez, H., Kohr, W.J. and Harkins, R.N. Anal. Biochem. 140, 538-547 (1984).
62. Hewick, R.M., Hunkapiller, M.W., Hood, L.E. and Dreyer, W.J. J. Biol. Chem. 256, 7990-7997 (1981).
63. Crea, R. and Horn, T. Nucl. Acids Res. 8, 2331-2348 (1980).
64. Beaucage, S.L. and Caruthers, M. Tetrahedron Lett. 22, 1859 (1981).
65. Ullrich, A., Berman, C.H., Dull, T.J., Gray, A. and Lee, J.M. EMBO J. 3, 361-364 (1984).
66. Benton, W.D. and Davis, R.W. Science 196, 180-182 (1977).
67. Kafatos, F.C., Jones, C.W. and Efstratiadis, A. Nucl. Acids Res. 7, 1541-1552 (1979).
68. Southern, E.M. J. Mol. Biol. 98, 503-517 (1975).
69. Sanger, F., Nicklen, S. and Coulson, A.R. Proc. Natl. Acad. Sci. USA 74, 5463-5467 (1977).
70. Messing, J., Crea, R. and Seeburg, P.H. Nucl. Acids Res. 9, 309-321 (1981).
71. Ullrich, A., Shine, J., Chirgwin, J., Pictet, R., Tischer, E., Rutter, W.J. and Goodman, H.M. Science 196, 1313-1317 (1977).
72. Aviv, H. and Leder, P. Proc. Natl. Acad. Sci. USA 69, 1408-1412 (1972).
73. Wickens, M.P., Buell, G.N. and Schimke, R.T. J. Biol. Chem. 253, 2483-2495 (1978).
74. Huynh, T.V., Young, R.A. and Davis, R.W. in DNA Cloning Techniques, A Practical Approach (Glover, D., ed.). (IRL, Oxford) pp 49-78 (1985).
75. Norris, K.E., Iserentant, D., Contreras, R. and Friers, W. Gene 7, 355-362 (1979).
76. Taylor, J.M., Illmensee, R. and Summers, S. Biochim. Biophys. Acta 442, 324-330 (1976).
77. Crowley et al., Molec. Cell. Biol. 3, 44-55 (1983).
78. Anzano, et al., Mol. Cell. Biol. 5, 242-247 (1985).
79. EP 73,656A

## Claims (Claims for the following Contracting State(s): BE, CH, DE, FR, GB, IT, LI, LU, NL, SE)

1. A method comprising (a) constructing a vector which includes nucleic acid encoding preTGF-β having the amino acid sequence shown in Figure 1b, or a mutant sequence thereof, (b) transforming a host vertebrate cell with the vector, (c) culturing the transformed cell, and (d) recovering mature TGF-β or mutant thereof from the culture medium, said TGF-β or mutant thereof having anchorage independent growth-promoting biological activity and cross-reacting with antisera raised against native TGF-β.

2. The method of claim 1 wherein the vertebrate cell is a Chinese hamster ovary cell line.

3. The method of claim 1 or claim 2 wherein the nucleic acid encoding the preTGF-β is operably linked to an inducible promoter.

4. The method of claim 1 or claim 2 wherein the nucleic acid encoding the preTGF-β is operably linked to a viral promoter.

5. The method of claim 4 wherein the promoter is an SV40 promoter.

6. The method of any one of claims 1 to 5 wherein the TGF-β is human TGF-β.

7. DNA encoding TGF-β having the amino acid sequence shown in Figure 1b, which DNA is free of one or more introns present in genomic DNA.

8. Extrachromosomal DNA encoding TGF-β having the amino acid sequence shown in Figure 1b.

9. mRNA encoding TGF-β of a given species having the amino acid sequence shown in Figure 1b, which mRNA is cell-free and free of mRNA encoding other proteins of the given species.

10. The DNA or mRNA of any one of claims 7 to 9 which is labelled with a detectable moiety.

11. An expression vector useful in the method of claim 1 comprising DNA that encodes preTGF-β having the amino acid sequence shown in Figure 1b or a mutant sequence thereof, whereby upon expression of said preTGF-β or mutant thereof, mature TGF-β or mutant thereof is produced having anchorage independent growth-promoting activity and cross-reacting with antisera raised against native TGF-β.

12. The vector of claim 11 wherein the DNA that encodes preTGF-β or a mutant sequence thereof is free of introns.

13. A host cell containing the vector of claim 11 or 12.

14. The cell of claim 13 which is a vertebrate cell.

15. The cell of claim 13 which is a bacterial cell.

## Claims (Claims for the following Contracting State(s): AT)

1. A method comprising (a) constructing a vector which includes nucleic acid encoding preTGF-β having the amino acid sequence shown in Figure 1b, or a mutant sequence thereof, (b) transforming a host vertebrate cell with the vector, (c) culturing the transformed cell, and (d) recovering mature TGF-β or mutant thereof from the culture medium, said TGF-β or mutant thereof having anchorage independent growth-promoting biological activity and cross-reacting with antisera raised against native TGF-β.

2. The method of claim 1 wherein the vertebrate cell is a Chinese hamster ovary cell line.

3. The method of claim 1 or claim 2 wherein the nucleic acid encoding the preTGF-β is operably linked to an inducible promoter.

4. The method of claim 1 or claim 2 wherein the nucleic acid encoding the preTGF-β is operably linked to a viral promoter.

5. The method of claim 4 wherein the promoter is an SV40 promoter.

6. The method of any one of claims 1 to 5 wherein the TGF-β is human TGF-β.

7. A method comprising the production of DNA encoding TGF-β having the amino acid sequence shown in Figure 1b, which DNA is free of one or more introns present in genomic DNA.

8. A method comprising the production of extrachromosomal DNA encoding TGF-β having the amino acid sequence shown in Figure 1b.

9. A method comprising the production of mRNA encoding TGF-β of a given species having the amino acid sequence shown in Figure 1b, which mRNA is cell-free and free of mRNA encoding other proteins of the given species.

10. A method according to any one of claims 7 to 9 wherein the DNA or mRNA is labelled with a detectable moiety.

11. A method comprising the production of an expression vector useful in the method of claim 1 comprising DNA that encodes preTGF-β having the amino acid sequence shown in Figure 1b or a mutant sequence thereof, whereby upon expression of said preTGF-β or mutant thereof, mature TGF-β or mutant thereof is produced having anchorage independent growth-promoting activity and cross-reacting with antisera raised against native TGF-β.

12. A method according to claim 11 wherein the DNA that encodes preTGF-β or a mutant sequence thereof is free of introns.

13. A method which comprises the preparation of a host cell containing the vector of claim 11 or 12.

14. The method of claim 13 wherein the host cell is a vertebrate cell.

15. The method of claim 13 wherein the host cell is a bacterial cell.

## Patentansprüche (Patentansprüche für folgende(n) Vertragsstaat(en): BE, CH, DE, FR, GB, IT, LI, LU, NL, SE)

1. Verfahren, umfassend (a) das Konstruieren eines Vektors, der Nukleinsäure enthält, die für preTGF-β mit der in Fig. 1b gezeigten Aminosäuresequenz, oder eine Mutantensequenz davon kodiert, (b) das Transformieren einer Wirtswirbeltierzelle mit dem Vektor, (c) das Kultivieren der transformierten Zelle und (d) das Rückgewinnen von reifem TGF-β oder eines Mutanten davon aus dem Kulturmedium, wobei das genannte TGF-β oder der Mutant davon eine verankerungsunabhängige wachstumsfördernde biologische Wirkung aufweist und mit gegen natives TGF-β gezüchtete Antiseren über Kreuz reagiert.

2. Verfahren nach Anspruch 1, worin die Wirbeltierzelle eine Eierstockzellinie vom Chinesischen Hamster ist.

3. Verfahren nach Anspruch 1 oder 2, worin die für das preTGF-β kodierende Nukleinsäure operabel mit einem induzierbaren Promotor verbunden ist.

4. Verfahren nach Anspruch 1 oder 2, worin die für das preTGF-β kodierende Nukleinsäure operabel mit einem viralen Promotor verbunden ist.

5. Verfahren nach Anspruch 4, worin der Promotor ein SV40-Promotor ist.

6. Verfahren nach einem der Ansprüche 1 bis 5, worin das TGF-β menschliches TGF-β ist.

7. DNA, die für TGF-β mit der in Fig. 1b gezeigten Aminosäuresequenz kodiert, welche DNA frei von einem oder mehreren Intronen ist, die in genomischer DNA vorhanden sind.

8. Extrachromosomale DNA, die für TGF-β mit der in Fig. 1b gezeigten Aminosäuresequenz kodiert.

9. mRNA, die für TGF-β einer bestimmten Spezies mit der in Fig. 1b gezeigten Aminosäuresequenz kodiert, welche mRNA zellfrei und frei von mRNA ist, die für andere Proteine der bestimmten Spezies kodiert.

10. DNA oder mRNA nach einem der Ansprüche 7 bis 9, die mit einer nachweisbaren Gruppe markiert ist.

11. Ein für das Verfahren nach Anspruch 1 nützlicher Expressionsvektor, der DNA umfaßt, die für preTGF-β mit der in Fig. 1b gezeigten Aminosäuresequenz oder einer Mutantensequenz davon kodiert, wodurch nach dem Exprimieren des genannten preTGF-β oder Mutanten davon reifes TGF-β oder Mutant davon erzeugt wird, das/der verankerungsunabhängige wachstumsfördernde Wirkung aufweist und mit gegen natives TGF-β gezüchtete Antiseren über Kreuz reagiert.

12. Vektor nach Anspruch 11, worin die DNA, die für preTGF-β oder eine Mutantensequenz davon kodiert, frei von Intronen ist.

13. Wirtszelle, die den Vektor nach Anspruch 11 oder 12 enthält.

14. Zelle nach Anspruch 13, die eine Wirbeltierzelle ist.

15. Zelle nach Anspruch 13, die eine Bakterienzelle ist.

## Patentansprüche (Patentansprüche für folgende(n) Vertragsstaat(en): AT)

1. Verfahren, umfassend (a) das Konstruieren eines Vektors, der Nukleinsäure enthält, die für preTGF-β mit der in Fig 1b gezeigten Aminosäuresequenz, oder eine Mutantensequenz davon kodiert, (b) das Transformieren einer Wirtswirbeitierzelle mit dem Vektor, (c) das Kultivieren der transformierten Zelle und (d) das Rückgewinnen von reifem TGF-β oder eines Mutanten davon aus dem Kulturmedium, wobei das genannte TGF-β oder der Mutant davon eine verankerungsunabhängige wachstumsfordernde biologische Wirkung aufweist und mit gegen natives TGF-β gezüchtete Antiseren über Kreuz reagiert.

2. Verfahren nach Anspruch 1, worin die Wirbeltierzelle eine Eierstockzellinie vom Chinesischen Hamster ist.

3. Verfahren nach Anspruch 1 oder 2, worin die für das preTGF-β kodierende Nukleinsäure operabel mit einem induzierbaren Promotor verbunden ist.

4. Verfahren nach Anspruch 1 oder 2, worin die für das preTGF-β kodierende Nukleinsäure operabel mit einem viralen Promotor verbunden ist.

5. Verfahren nach Anspruch 4, worin der Promotor ein SV40-Promotor ist.

6. Verfahren nach einem oder Ansprüche 1 bis 5, worin das TGF-β menschliches TGF-β ist.

7. Verfahren, welches die Herstellung von DNA umfaßt, die für TGF-β mit der in Fig. 1b gezeigten Aminosäuresequenz kodiert, welche DNA frei von einem oder mehreren Intronen ist, die in genomischer DNA vorhanden sind.

8. Verfahren, welches die Herstellung von extrachromosomaler DNA umfaßt, die für TGF-β mit der in Fig. 1b gezeigten Aminosäuresequenz kodiert.

9. Verfahren, welches die Herstellung von mRNA umfaßt, die für TGF-β einer bestimmten Spezies mit der in Fig. 1b gezeigten Aminosäuresequenz kodiert, welche mRNA zellfrei und frei von mRNA ist, die für andere Proteine der bestimmten Spezies kodiert.

10. Verfahren nach einem der Ansprüche 7 bis 9, worin die DNA oder mRNA mit einer nachweisbaren Gruppe markiert wird.

11. Verfahren, welches die Herstellung eines für das Verfahren nach Anspruch 1 nützlichen Expressionsvektors umfaßt, der DNA umfaßt, die für preTGF-β mit der in Fig. 1b gezeigten Aminosäuresequenz oder eine Mutantensequenz davon kodiert, wodurch nach dem Exprimieren des genannten preTGF-β oder Mutanten davon reifes TGF-β oder Mutant davon erzeugt wird, das/der verankerungsunabhängige wachstumsfördernde Wirkung aufweist und mit gegen natives TGF-β gezüchtete Antiseren über Kreuz reagiert.

12. Verfahren nach Anspruch 11, worin die DNA, die für preTGF-β oder eine Mutantensequenz davon kodiert, frei von Intronen ist.

13. Verfahren, welches die Herstellung einer Wirtszelle umfaßt, die den Vektor nach Anspruch 11 oder 12 enthält.

14. Verfahren nach Anspruch 13, worin die Wirtszelle eine Wirbeltierzelle ist.

15. Verfahren nach Anspruch 13, worin die Wirtszelle eine Bakterienzelle ist.

## Revendications (Revendications pour l'(les) Etat(s) contractant(s) suivant(s): BE, CH, DE, FR, GB, IT, LI, LU, NL, SE)

1. Procédé comprenant (a) la construction d'un vecteur qui comprend un acide nucléique codant pour un préTGF-β ayant la séquence d'amino-acides représentée sur la figure 1b, ou une de ses séquences mutantes, (b) la transformation d'une cellule-hôte de vertébré avec le vecteur, (c) la culture de la cellule transformée, et (d) la séparation du TGF-β mature ou d'un de ses mutants du milieu de culture, ledit TGF-β ou son mutant possédant une activité biologique d'activation de croissance indépendante de l'ancrage et une réactivité croisée avec des anti-sérums engendrés contre le TGF-β naturel.

2. Procédé suivant la revendication 1, dans lequel les cellules de vertébré consistent en une lignée de cellules d'ovaire d'hamster chinois.

3. Procédé suivant la revendication 1 ou la revendication 2, dans lequel l'acide nucléique codant pour le préTGF-β est lié de manière fonctionnelle à un promoteur inductible.

4. Procédé suivant la revendication 1 ou la revendication 2, dans lequel l'acide nucléique codant pour le préTGF-β est lié de manière fonctionnelle à un promoteur viral.

5. Procédé suivant la revendication 4, dans lequel le promoteur est un promoteur de SV40.

6. Procédé suivant l'une quelconque des revendications 1 à 5, dans lequel le TGF-β est le TGF-β humain.

7. Procédé comprenant la production d'un ADN codant pour un TGF-β ayant la séquence d'amino-acides représentée sur la figure 1b, ADN qui est dépourvu d'un ou plusieurs introns présents dans l'ADN génomique.

8. ADN extrachromosomique codant pour un TGF-β ayant la séquence d'amino-acides représentée sur la figure 1b.

9. ARNm codant pour un TGF-β d'une espèce donnée ayant la séquence d'amino-acides représentée sur la figure 1b, l'ARNm étant acellulaire et dépourvu d'un ARNm codant pour d'autres protéines de l'espèce donnée.

10. L'ADN ou l'ARNm suivant l'une quelconque des revendications 7 à 9, qui est marqué avec un groupement détectable.

11. Vecteur d'expression utile dans le procédé suivant la revendication 1, comprenant un ADN qui code pour un préTGF-β ayant la séquence d'amino-acides représentée sur la figure 1b ou une de ses séquences mutantes, permettant, par expression dudit préTGF-β ou de son mutant, la production d'un TGF-β mature ou de son mutant possédant une activité d'activation de croissance indépendante de l'ancrage et une réactivité croisée avec des anti-sérums engendrés contre le TGF-β naturel.

12. Vecteur suivant la revendication 11, dans lequel l'ADN qui code pour le préTGF-β ou une de ses séquences mutantes est dépourvu d'introns.

13. Cellule-hôte contenant le vecteur suivant la revendication 11 ou 12.

14. Cellule suivant la revendication 13, qui est une cellule de vertébré.

15. Cellule suivant la revendication 13, qui est une cellule bactérienne.

## Revendications (Revendications pour l'(les) Etat(s) contractant(s) suivant(s): AT)

1. Procédé comprenant (a) la construction d'un vecteur qui comprend un acide nucléique codant pour un préTGF-β ayant la séquence d'amino-acides représentée sur la figure 1b, ou une de ses séquences mutantes, (b) la transformation d'une cellule-hôte de vertébré avec le vecteur, (c) la culture de la cellule transformée, et (d) la séparation du TGF-β mature ou d'un de ses mutants du milieu de culture, ledit TGF-β ou son mutant possédant une activité biologique d'activation de croissance indépendante de l'ancrage et une réactivité croisée avec des anti-sérums engendrés contre le TGF-β naturel.

2. Procédé suivant la revendication 1, dans lequel les cellules de vertébré consistent en une lignée de cellules d'ovaire d'hamster chinois.

3. Procédé suivant la revendication 1 ou la revendication 2, dans lequel l'acide nucléique codant pour le préTGF-β est lié de manière fonctionnelle à un promoteur inductible.

4. Procédé suivant la revendication 1 ou la revendication 2, dans lequel l'acide nucléique codant pour le préTGF-β est lié de manière fonctionnelle à un promoteur viral.

5. Procédé suivant la revendication 4, dans lequel le promoteur est un promoteur de SV40.

6. Procédé suivant l'une quelconque des revendications 1 à 5, dans lequel le TGF-β est le TGF-β humain.

7. Procédé comprenant la production d'un ADN codant pour un TGF-β ayant la séquence d'amino-acides représentée sur la figure 1b, ADN qui est dépourvu d'un ou plusieurs introns présents dans l'ADN génomique.

8. Procédé comprenant la production d'un ADN extrachromosomique codant pour un TGF-β ayant la séquence d'amino-acides représentée sur la figure 1b.

9. Procédé comprenant la production d'un ARNm codant pour un TGF-β d'une espèce donnée ayant la séquence d'amino-acides représentée sur la figure 1b, l'ARNm étant acellulaire et dépourvu d'un ARNm codant pour d'autres protéines de l'espèce donnée.

10. Procédé suivant l'une quelconque des revendications 7 à 9, dans lequel l'ADN ou l'ARNm est marqué avec un groupement détectable.

11. Procédé comprenant la production d'un vecteur d'expression utile dans le procédé suivant la revendication 1, comprenant un ADN qui code pour un préTGF-β ayant la séquence d'amino-acides représentée sur la figure 1b ou une de ses séquences mutantes, permettant, par expression dudit préTGF-β ou de son mutant, la production d'un TGF-β mature ou de son mutant possédant une activité d'activation de croissance indépendante de l'ancrage et une réactivité croisée avec des anti-sérums engendrés contre le TGF-β naturel.

12. Procédé suivant la revendication 11, dans lequel l'ADN qui code pour le préTGF-β ou une de ses séquences mutantes est dépourvu d'introns.

13. Procédé qui comprend la préparation d'une cellule-hôte contenant le vecteur suivant la revendication 11 ou 12.

14. Procédé suivant la revendication 13, dans lequel la cellule-hôte est une cellule de vertébré.

15. Procédé suivant la revendication 13, dans lequel la cellule-hôte est une cellule bactérienne.
